# EUROPÄISCHE PATENTSCHRIFT

(11) **EP 1 497 030 B1**
(45) Veröffentlichungstag und Bekanntmachung des Hinweises auf die Patenterteilung: **13.09.2006**
(21) Anmeldenummer: 03720392.4
(22) Anmeldetag: 28.03.2003
(51) Int. Cl.: B01L 3/00

(54) **VORRICHTUNG AUF BASIS VON PARTIELL OXIDIERTEM PORÖSEN SILIZIUM UND VERFAHREN ZU DEREN HERSTELLUNG**
DEVICE BASED ON PARTIALLY OXIDIZED POROUS SILICON AND METHOD FOR THE PRODUCTION THEREOF
DISPOSITIF A BASE DE SILICIUM POREUX PARTIELLEMENT OXYDE ET SON PROCEDE DE PRODUCTION

(30) Priorität: 19.04.2002 DE 10217569
(43) Veröffentlichungstag der Anmeldung: 19.01.2005
(73) Patentinhaber: Infineon Technologies AG, 81669 München (DE)
(72) Erfinder: DERTINGER, Stephan, 80335 München (DE); FRITZ, Michaela, 81673 München (DE); FUCHS, Karin, 81739 München (DE); HANEDER, Thomas, 85221 Dachau (DE); LEHMANN, Volker, 80689 München (DE); MARTIN, Alfred, 81825 München (DE); MÄRZ, Reinhard, 81477 München (DE)
(74) Vertreter: Müller-Boré & Partner Patentanwälte
(86) Internationale Anmeldenummer: PCT/EP2003/003293
(87) Internationale Veröffentlichungsnummer: WO 2003/089925

(56) Entgegenhaltungen:
- WO-A-01/24929
- DE-A- 4 426 507
- US-A- 3 962 052
- US-A- 5 987 208
- US-A1- 2002 191 884
- MASINI G ET AL: "Si based optoelectronics for communications" MATERIALS SCIENCE AND ENGINEERING B, ELSEVIER SEQUOIA, LAUSANNE, CH, Bd. 89, Nr. 1-3, 14. Februar 2002 (2002-02-14), Seiten 2-9, XP004334357 ISSN: 0921-5107
- BIRNER A ET AL: "SILICON-BASED PHOTONIC CRYSTALS" ADVANCED MATERIALS, VCH VERLAGSGESELLSCHAFT, WEINHEIM, DE, Bd. 13, Nr. 6, 16. März 2001 (2001-03-16), Seiten 377-388, XP001039026 ISSN: 0935-9648

## Beschreibung

Die vorliegende Erfindung betrifft eine Vorrichtung, umfassend ein flächig ausgebildetes makroporöses Trägermaterial auf Silizium-Basis, das über mindestens einen Oberflächenbereich verteilt eine Vielzahl von Poren mit einem Durchmesser im Bereich von 500 nm bis 100 µm aufweist, welche sich von einer Oberfläche zur gegenüberliegenden Oberfläche des Trägermaterials durchgängig erstrecken, wobei die Vorrichtung mindestens einen Bereich aufweist, der mehrere Poren mit Porenwänden aus SiO₂ umfasst, und wobei dieser Bereich von einem zu den Längsachsen der Poren im wesentlichen parallel angeordneten, zu den Oberflächen des Trägermaterials hin offenen Rahmen aus Wänden mit einem Siliziumkern umgeben ist, wobei der Kern aus Silizium über den Querschnitt zur Außenseite der den Rahmen bildenden Wände hin in Siliziumdioxid übergeht, sowie ein Verfahren zu deren Herstellung. Die erfindungsgemäße Vorrichtung eignet sich als Basis für ein "BioChip-Grundmodul" in Verfahren zum Nachweis biochemischer (Bindungs)Reaktionen sowie hierfür insbesondere zur Untersuchung von enzymatischen Reaktionen, Nukleinsäure-Hybridisierungen, Protein-Protein-Wechselwirkungen und anderer Bindungsreaktionen im Bereich der Genom-, Proteom- oder Wirkstoff-Forschung in Biologie und Medizin.

In der Molekularbiologie finden heute in zunehmendem Maße Biochips Verwendung, mit denen auf schnelle Art und Weise Erkenntnisse über Organismen und Gewebe gewonnen werden. Für die Biowissenschaften und die medizinische Diagnostik ist die Detektion (bio)chemischer Reaktionen, d.h. die Detektion biologisch relevanter Moleküle in definiertem Untersuchungsmaterial von herausragender Bedeutung. In diesem Rahmen wird die Entwicklung von sogenannten BioChips stetig vorangetrieben. Bei derartigen BioChips handelt es sich üblicherweise um miniaturisierte hybride Funktionselemente mit biologischen und technischen Komponenten, insbesondere auf einer Oberfläche eines BioChip-Grundmoduls immobilisierten Biomolekülen, die als spezifische Interaktionspartner dienen. Häufig weist die Struktur dieser Funktionselemente Reihen und Spalten auf. Man spricht dann von sogenannten "Mikroarrays". Da tausende von biologischen bzw. biochemischen Funktionselementen auf einem Chip angeordnet sein können, werden diese in der Regel mit mikrotechnischen Methoden angefertigt.

Als biologische und biochemische Funktionselemente kommen insbesondere DNA, RNA, PNA, (bei Nukleinsäuren und ihren chemischen Derivaten können z.B. Einzelstränge wie Oligonukleotide, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), einzelne Zellen, mehrzellige Organismen sowie Zellverbände in Frage.

Die am weitesten verbreitete Variante von Biochips sind die sogenannten Microarrays. Dies sind kleine Plättchen ("Chips") aus beispielsweise Glas, Gold, Kunstoff oder Silizium. Zum Nachweis entsprechender biologischer oder biochemischer (Bindungs)Reaktionen werden beispielsweise kleine Mengen an solubilisierten unterschiedlichen Fängermolekülen, z.B. eine bekannte Nukleinsäuresequenz, in Form von kleinsten Tröpfchen punktförmig und matrizenartig, sogenannte Dots, auf der Oberfläche des BioChip-Grundmoduls fixiert.

In der Praxis werden einige hundert bis einige tausend Tröpfchen pro Chip verwendet. Anschließend wird ein zu untersuchender Analyt, der beispielsweise fluoreszenzmarkierte Zielmoleküle enthalten kann, über diese Oberfläche gepumpt. Dabei kommt es im allgemeinen zu unterschiedlichen chemischen (Bindungs)Reaktionen zwischen den im Analyt enthaltenen Zielmolekülen und den fixierten bzw. immobilisierten Fängermolekülen. Wie bereits angeführt, werden zur Beobachtung dieser Reaktionen oder Bindungen die Zielmoleküle mit Farbstoffmolekülbausteinen, üblicherweise Fluorochromen markiert. Das Vorhandensein und die Intensität von Licht, das von den Fluorochromen emittiert wird, gibt Aufschluß über den Verlauf der Reaktion oder Bindung in den einzelnen Tröpfchen auf dem Substrat, so daß Rückschlüsse auf das Vorhandensein und/oder die Eigenschaft der Zielmoleküle und/oder Fängermoleküle gezogen werden können. Wenn sich die entsprechenden fluoreszenzmarkierten Zielmoleküle des Analyten mit bzw. an den an der Oberfläche des Trägersubstrats immobilisierten Fängermolekülen umsetzen bzw. binden, kann durch optische Anregung mit einem Laser und Messung des entsprechenden Fluoreszenzsignals diese Reaktion bzw. Bindung nachgewiesen werden.

Substrate mit hoher, aber definierter Porosität weisen als Basis für derartige BioChips mehrere Vorteile gegenüber planaren Substraten auf. Auf der stark vergrößerten Oberfläche können mehr Nachweisreaktionen stattfinden. Dadurch steigt die Nachweisempfindlichkeit für biologische Assays. Durch Pumpen der im Analyt gelösten Zielmoleküle durch die Kanäle zwischen Vorder- und Rückseite des porösen Substrates werden diese in nahen räumlichen Kontakt mit der Oberfläche des Substrates gebracht (< 10 µm). Auf dieser Größenskala ist die Diffusion ein sehr effektiver Transportprozess, der innerhalb kurzer Zeit die Distanzen zwischen nachzuweisendem Zielmolekül und dem auf der Oberfläche immobilisierten Fängermolekül überbrückt. Die Geschwindigkeit der Bindungsreaktion kann dadurch erhöht und damit die Dauer des Nachweisverfahrens deutlich verkürzt werden.

Ein Beispiel für ein Substrat mit derartiger definierter Porösität ist elektrochemisch hergestelltes poröses Silizium (vgl. DE 42 02 454 A1, EP 0 553 465 A1 oder DE 198 20 756 A1).

Ein großer Teil der heute verwendeten analytischen Methoden in der Wirkstoff-Forschung und klinischen Diagnostik setzt optische Verfahren zum Nachweis von Bindungsereignissen zwischen nachzuweisender Substanz und Fängermolekülen ein (z.B. DNA-Hybridisierungen, Antikörper-Antigen-Wechselwirkungen und Protein-Wechselwirkungen). Die nachzuweisende Substanz wird hierbei mit einem Marker versehen, der nach Anregung mit Licht geeigneter Wellenlänge fluoresziert (Fluoreszenzverfahren) oder der eine chemische Reaktion auslöst, die wiederum Licht erzeugt (Chemilumineszenzverfahren). Bindet die nachzuweisende Substanz, d.h. das Zielmolekül, mit dem immobilisierten Fängermolekül auf der Oberfläche, so kann dies optisch, z.B. über Lumineszenz, nachgewiesen werden. Unter dem Begriff "Lumineszenz" wird hierbei die spontane Emission von Photonen im ultravioletten bis infraroten Spektralbereich bezeichnet. Anregungsmechanismen der Lumineszenz können optischer oder nicht-optischer Natur sein, beispielsweise elektrische, chemische, biochemische und/oder thermische Anregungsprozesse. Somit sollen insbesondere Chemi-, Bio- und Elektrolumineszenz sowie Fluoreszenz und Phosphoreszenz unter den Begriff "Lumineszenz" im Sinne dieser Erfindung fallen.

Poröse Substrate mit hoher optischer Dichte und geringer Reflektivität, wie beispielsweise poröses Silizium, dessen Reflektivität im sichtbaren Bereich des Spektrums 50 bis 70 % beträgt, liefern in Verbindung mit Fluoreszenz- oder Chemilumineszenzverfahren jedoch nicht die erwarteten Ergebnisse, insofern die experimentell beobachtete Lichtsignalausbeute bei weitem nicht an die theoretisch erreichbaren Werte heranreicht. Der Grund für die gegenüber den theoretischen Werten verringerte, experimentell beobachtete Lichtsignalausbeute beim Einsatz derartiger poröser Substrate liegt zum einen in Problemen bei der Emission der Fluoreszenz der zu untersuchenden Substanz bzw. Bindung und zum anderen - wenn ein Fluoreszenzverfahren vorliegt - in Problemen bei der optischen Anregung der Fluoreszenz begründet.

Wird im gesamten Volumen der Poren (Luminenszenz-)Licht erzeugt, spielt die Reflektivität der Porenwände eine entscheidende Rolle für die effektive Herausleitung des optischen Signals zur Oberfläche. Im Falle der Chemilumineszenz wird das Lichtsignal isotrop in alle Raumrichtungen abgestrahlt. Infolgedessen strahlt nur ein sehr geringer Anteil des erzeugten Lichts direkt im Öffnungswinkel der einzelnen Pore ab. Alle anderen Strahlengänge werden an den Wänden der Poren mehrfach reflektiert, bis sie die Öffnung der jeweiligen Pore erreichen. Schon bei Reflektivitäten, die nur wenig unterhalb von 100 % liegen, ist jedoch die Intensität eines Signals nach mehreren Reflexionen stark vermindert. Dieser Effekt hat zur Folge, daß dieser Anteil des erzeugten Signals auf dem Weg aus der Pore stark abgeschwächt wird und kaum noch zum Gesamtsignal beitragen kann.

Ferner stellt auch die Abschwächung durch Vielfachreflektionen an den Porenwänden, welche bereits im Zusammenhang mit den Problemen bei der Anregung der Fluoreszenz beschrieben worden ist, bei der Emission der Lumineszenz ein ernstzunehmendes Problem dar. Nur Fluorophore (fluoreszierende Substanzen im Analyten), welche direkt in Richtung der Porenöffnung abstrahlen, stehen unabgeschwächt für ein Fluoreszenzsignal zur Verfügung. Alle übrigen Strahlengänge werden an den Wänden der Poren mindestens einmal reflektiert bis sie die Öffnung der Pore erreichen. Schon bei Reflektivitäten, welche nur wenig unterhalb von 100% liegen, führen diese Vielfachreflektionen zu einer empfindlichen Abschwächung des zu detektierenden optischen Signals.

Um die oben genannten Probleme der Intensitätsabschwächung durch Vielfachreflektionen zu lösen, wurde vorgeschlagen, Reflexschichten zu einer Verringerung der Reflektionsverluste an den Porenwänden anzuordnen, um dadurch das Anregungs- und Emissionslicht besser aus der Poren herausleiten zu können. Jedoch konnte dieser Lösungsansatz keine signifikante Verbesserung der Signalausbeute bewirken.

Der vorliegenden Erfindung liegt somit die Aufgabe zugrunde, eine Vorrichtung bzw. ein "BioChip-Grundmodul" zum Nachweis biochemischer Reaktionen und/oder Bindungen bereitzustellen, die bzw. das im Rahmen von Analyseverfahren auf Fluoreszenz- oder Chemilumineszenz-Basis eine höhere absolute Signalausbeute bei verbessertem Signal-Rausch-Verhältnis liefern soll, um so die Nachweisempfindlichkeit von mit dem fertigen Biochip durchzuführenden Tests zu steigern.

Diese Aufgabe wird durch die in den Ansprüchen gekennzeichneten Ausführungsformen gelöst.

Insbesondere wird eine Vorrichtung bereitgestellt, umfassend ein flächig ausgebildetes makroporöses Trägermaterial (10) auf Silizium-Basis, das über mindestens einen Oberflächenbereich verteilt eine Vielzahl von periodisch angeordneten, diskreten Poren (11) mit einem Durchmesser im Bereich von 500 nm bis 100 µm aufweist, welche sich von einer Oberfläche (10A) zur gegenüberliegenden Oberfläche (10B) des Trägermaterials durchgängig erstrecken, wobei die Vorrichtung mindestens einen Bereich (11A) aufweist, der mehrere Poren mit Porenwänden aus SiO₂ umfasst, und wobei dieser Bereich von einem zu den Längsachsen der Poren im wesentlichen parallel angeordneten, zu den Oberflächen (10A, 10B) hin offenen Rahmen (12) aus Wänden mit einem Siliziumkern (12A) umgeben ist, wobei der Kern aus Silizium über den Querschnitt zur Außenseite der den Rahmen bildenden Wände hin in Siliziumdioxid übergeht.

Die erfindungsgemäße Vorrichtung weist lokal vollständig durchoxidierte Bereiche aus SiO₂ auf, d.h. Bereiche, die mehrere Poren mit Porenwänden aus SiO₂ umfassen. Diese durchoxidierten Bereiche sind wiederum von einer Überstruktur umgeben. Die durchoxidierten Bereiche sind von Wänden aus im wesentlichen Silizium eingefasst bzw. umgeben, so daß diese Wände aus im wesentlichen Silizium einen zu den Oberflächen (10A, 10B) hin offenen Rahmen bzw. Zylinder, dessen Zylinderachse parallel zu den Poren verläuft, bilden, der die lokal vollständig durchoxidierten Bereiche aus SiO₂ umgibt bzw. umschließt. Die den Rahmen bildenden Wände weisen einen Kern aus Silizium auf, wobei, über einen in der Flächenebene des Trägermaterials verlaufenden Querschnitt gesehen, das Silizium zur Außenseite der Wände hin in Siliziumdioxid übergeht. Der Rahmen bzw. die Überstruktur kann beliebig gestaltet sein. Gemäß der vorliegenden Erfindung kann der Rahmen (12) auch als Teilrahmen nach einer oder mehreren Seiten offen sein, d.h. eine oder mehrere der den Rahmen bildenden Wände fehlt.

In den durchoxidierten Bereichen sind die Wände zwischen den Poren vollständig aus SiO₂ aufgebaut. Diese Bereiche sind damit für Wellenlängen insbesondere im sichtbaren Bereich transparent. Die erfindungsgemäße Vorrichtung weist somit lokal transparente Bereichen aus SiO₂ auf, wobei diese transparenten Bereiche wiederum von einem reflektierenden Rahmen aus Wänden mit Siliziumkern umgeben sind. Mit anderen Worten, es liegen lokal vollständig transparente Bereiche aus SiO₂ vor, die durch nicht-transparente Wände mit Siliziumkern, welche in der erfindungsgemäßen Vorrichtung quasi eine Sekundärstruktur bilden, voneinander getrennt sind.

Der Rahmen (12) aus Wänden mit einem Siliziumkern, der zu den beiden Außenseiten hin jeweils in Siliziumdioxid übergeht, eliminiert Streulicht und optisches Übersprechen zwischen den Bereichen, die mehrere Poren mit Porenwänden aus SiO₂ umfassen. Dies ist ein wesentlicher Vorteil gegenüber porösen Substraten, die vollständig transparent sind (z.B. SiO₂, Glaschips oder Al₂O₃).

In der erfindungsgemäßen Vorrichtung ist über mindestens einen Oberflächenbereich des flächig ausgebildeten makroporösen Trägermaterials (10) verteilt eine Vielzahl von üblicherweise periodisch angeordneten Poren, welche sich von einer Oberfläche (10A) zur gegenüberliegenden Oberfläche (10B) des Trägermaterials erstrecken, angeordnet. Im Rahmen der vorliegenden Erfindung können auf dem flächig ausgebildeten makroporösen Trägermaterial (10) bereichsweise auch Sacklöcher, d.h. nur nach einer der Oberflächenseiten (10A, 10B) geöffnete Poren, vorgesehen werden.

Das eingesetzte makroporöse Trägermaterial weist einen Porendurchmesser von 500 nm bis 100 µm, vorzugsweise 2 bis 10 µm auf. Die Dicke des makroporösen Trägermaterials beträgt üblicherweise 100 bis 5.000 µm, vorzugsweise 300 bis 600 µm. Der Abstand von Porenmitte zu Porenmitte (Pitch), d.h. zweier zueinander benachbarter bzw. angrenzender Poren beträgt üblicherweise 1 bis 500 µm, vorzugsweise 3 bis 100 µm. Die Porendichte liegt üblicherweise im Bereich von 10⁴ bis 10⁸/cm².

Die Poren (11) in der erfindungsgemäßen Vorrichtung können beispielsweise im wesentlichen rund oder ellipsenförmig gestaltet sein. In einer bevorzugten Ausführungsform der vorliegenden Erfindung sind die Poren (11) mit Porenwänden aus SiO₂ im wesentlichen quadratisch ausgebildet. Der Rahmen (12) aus Wänden mit einem Siliziumkern (12A) kann dann in im wesentlichen quadratischer oder rechteckiger Form vorliegen.

Ein weiterer Gegenstand der vorliegenden Erfindung betrifft ein Verfahren zur Herstellung einer wie vorstehend beschriebenen erfindungsgemäßen Vorrichtung, umfassend die Schritte:
(a) Bereitstellen eines Trägermaterials aus Silizium mit den Oberflächen (10A, 10B);
(b) Erzeugen von Sacklöchern, deren Tiefe geringer als die Dicke des Trägermaterials ist, durch elektrochemisches Ätzen in einer Oberfläche (10A) des Trägermaterials dergestalt, daß der Abstand der in einer ansonsten, im wesentlichen regelmäßigen Anordnung vorgesehenen Sacklöcher unter Ausbildung von Bereichsübergängen mit erhöhter Siliziumwanddicke bereichsweise geändert wird, wobei die Dicke der Siliziumwände zwischen den Bereichsübergängen um den Betrag des erhöhten Sacklöcherabstandes größer als die Dicke der Siliziumwände innerhalb der Bereiche ist;
(c) mindestens bereichsweises Anordnen einer Maskenschicht auf der Oberfläche (10A) und der Oberfläche der in Schritt (b) erzeugten Sacklöcher;
(d) Abtragen des Trägermaterials mindestens bis zum Boden der Sacklöcher unter Erhalten von Poren (11), welche sich von einer Oberfläche (10A) zur gegenüberliegenden Oberfläche (10B) des Trägermaterials durchgängig erstrecken;
(e) Entfernen der Maskenschicht;
(f) Unterwerfen des in Schritt (e) erhaltenen Trägermaterials einer thermischen Oxidation, so daß in Abhängigkeit von der Siliziumwanddicke die Bereiche mit dünneren Silizium-Wänden vollständig oxidiert werden, während bei den Bereichsübergängen mit erhöhter Wanddicke die Siliziumwände nicht vollständig oxidiert werden, so daß ein Siliziumkern in den Wänden bestehen bleibt.

Das in Schritt (a) bereitgestellte Trägermaterial aus Silizium kann beispielsweise n-dotiertes einkristallines Silizium (Si-Wafer) sein.

In Schritt (b) des erfindungsgemäßen Verfahrens erfolgt dann ein elektrochemischen Ätzen in das Silizium. Ein derartiges Verfahren ist beispielsweise aus EP 0 296 348, EP 0 645 621, WO 99/25026, DE 42 02 454, EP 0 553 465 oder DE 198 20 756 bekannt, auf die in vollem Umfang Bezug genommen wird und deren Offenbarungsgehalt insoweit Teil der vorliegenden Anmeldung sein soll. Im Rahmen eines derartigen elektrochemischen Ätzens können Sacklöcher bzw. Poren mit Aspektverhältnissen von beispielsweise 1 zu 300 und mehr in einer im wesentlichen regelmäßigen Anordnung in Silizium geätzt werden. Da das elektrochemische Porenätzverfahren bei geeignet gewählten Parametern eine Änderung des Porenabstandes (Pitch) in bestimmten Grenzen zulässt, kann durch Änderung des Porenabstandes oder das Weglassen einer ganzen Reihe von Poren in der ansonsten regelmäßigen Anordnung von Sacklöchern bzw. Poren die Dicke der resultierenden Silizium-Wände lokal verändert werden.

Um Poren zu erhalten, die durch das Trägermaterial bzw. Substrat (Si-Wafer) gehen und auf beiden Oberflächen (10A, 10B) geöffnet sind, wird in den Schritten (c), (d) und (e) nach der Ätzung der Sacklöcher auf der Rückseite des Si-Wafers z.B. durch eine KOH-Ätzung Silizium abgetragen, während die Vorderseite des Wafers und die Innenseite der Sacklöcher bzw. Poren durch eine Maskenschicht, wie z.B. eine durch CVD-Abscheidung erzeugte Siliziumnitridschicht in einer Dicke von beispielsweise 100 nm, geschützt werden. Die Maskenschicht kann dann in Schritt (e) beispielsweise mittels HF-Behandlung entfernt werden. Zum rückseitigen Abtragen des Si-Wafers eigenen sich gleichermaßen Sputter-, Laserablations und/oder Polierprozesse, beispielsweise ein CMP-Prozeß.

Dadurch wird ein Silizium-Wafer bzw. Silizium-Trägermaterial erzeugt, das matrixartig mit regelmäßigen Poren versehen ist, wobei die Poren durchgehende Röhren darstellen, welche die Vorder- und die Rückseite des Wafers miteinander verbinden.

Der Durchmesser dieser Poren kann nach deren Herstellung z.B. durch Ätzen in KOH vergrößert bzw. aufgeweitet werden. Wird Si(100) als Ausgangsmaterial verwendet, so entstehen bei einer solchen Ätzung auf Grund der Kristallstruktur im wesentlichen quadratische Poren. Geht man beispielsweise von Porendurchmesser von ca. 5 µm mit einem Abstand zwischen den Mittelpunkten zweier Poren (Pitch) von 12 µm aus, so kann dadurch der Porendurchmesser z.B. von 5 µm auf 10 bis 11 µm vergrößert werden. Die Dicke der Silizium-Wände zwischen den Poren nimmt dabei gleichzeitig auf 2 bis 1 µm ab. Auf diese Weise wird quasi ein quadratisches Gitter aus dünnen Silizium-Wänden erhalten. Die Tiefe der Poren bzw. die Länge der Silizium-Wände entspricht dabei der ursprünglichen Dicke des Silizium-Wafers, vermindert um die Dicke der beim Öffnen der Poren auf der Rückseite abgetragenen Si-Schicht.

In Schritt (f) wird das derart erhaltene Gitter in einem thermischen Oxidationsprozess z.B. bei einer Temperatur von 1100°C und einer Dauer von 6 Stunden durch Oxidation in Abhängigkeit von der jeweiligen Porenwanddicke in SiO₂ überführt. Die Struktur des Substrates wird dabei bis auf eine Volumenzunahme der Wandbereiche durch die Oxidation von Si zu SiO₂ im wesentlichen nicht verändert.

Wird in Schritt (b) der Abstand der Sacklöcher bzw. Poren voneinander periodisch, beispielsweise alle 5, 10 oder 20 Poren, geringfügig, z.B. um 1 µm, erhöht, so entsteht dadurch eine Überstruktur, die sich aus Bereichen mit Arrays aus Poren (beispielsweise 5 x 5, 10 x 10, 20 x 20) zusammensetzt. Die Dicke der Siliziumwände ist zwischen diesen Bereichen um den Betrag des erhöhten Porenabstandes größer als die Dicke der Siliziumwände innerhalb der Bereiche. Bei anschließender Oxidation in Schritt (f) werden die Bereiche mit dünnen Silizium-Wänden vollständig zu SiO₂ oxidiert. Bei den Übergängen zwischen den Bereichen, die eine erhöhte Wanddicke aufweisen, werden die Siliziumwände jedoch nicht vollständig oxidiert, so daß ein Siliziumkern in den Wänden bestehen bleibt, wobei der Kern aus Silizium über den Querschnitt zur Außenseite der den Rahmen bildenden Wände hin jeweils in Siliziumdioxid übergeht. Es entstehen dadurch lokal vollständig transparente Bereiche aus SiO₂, die durch nicht-transparente Wände mit Siliziumkern voneinander getrennt sind.

Unmittelbar daran kann dann das Anbringen bzw. Binden von Linkermolekülen erfolgen. Solche Linkermoleküle unterliegen keiner spezifischen Beschränkung, solange sie befähigt sind, an die auf der Oberfläche der SiO₂-Schicht vorliegenden OH-Gruppen kovalent zu binden und weiter eine funktionelle Gruppe aufweisen, die zur kovalenten Bindung mit als Sonden in biologisch-chemischen Reaktionen einsetzbaren Fängermolekülen befähigt ist. Solche Linkermoleküle sind üblicherweise auf der Basis einer Silicium-organischen Verbindung. Derartige bifunktionelle Silicium-organische Verbindungen können beispielsweise Alkoxysilan-Verbindungen mit einer oder mehreren terminalen funktionalen Gruppen, ausgewählt aus Epoxy, Glycidyl, Chlor, Mercapto oder Amino, sein. Vorzugsweise ist die Alkoxysilan-Verbindung ein Glycidoxyalkylalkoxysilan, wie z.B. 3-Glycidoxypropyltrimethoxysilan, ein Mercaptoalkylalkoxysilan, wie z.B. γ-Mercaptopropyltrimethoxysilan, oder ein Aminoalkylalkoxysilan, wie z.B. N-β-(aminoethyl) γ-aminopropyltrimethoxysilan. Die Länge der als Spacer zwischen der funktionellen Gruppe, wie z.B. Epoxy bzw. Glycidoxy, welche mit dem Fängermolekül bzw. der Sonde bindet, und der Trialkoxysilangruppe wirkenden Alkylenreste unterliegt dabei keiner Beschränkung. Derartige Spacer können auch Polyethylenglykolreste sein.

Zur Endfertigung eines BioChips kann dann das Anbinden bzw. Koppeln von Fängermolekülen wie beispielsweise Oligonukleotiden bzw. DNA-Molekülen an das Trägermaterial über die Linkermoleküle nach den im Stand der Technik üblichen Verfahren erfolgen, beispielsweise mittels Behandeln des porösen Trägermaterials bei Verwendung von Epoxysilanen als Linkermoleküle durch anschließende Reaktion der terminalen Epoxidgruppen mit terminalen primären Aminogruppen oder Thiolgruppen von Oligonukleotiden bzw. DNA-Molekülen, die in entsprechenden Analyseverfahren als immobilisierte bzw. fixierte Fängermoleküle für die im zu untersuchenden Analyten vorliegenden Zielmoleküle fungieren. Dabei können beispielsweise die als Fängermoleküle verwendbaren Oligonukleotide unter Verwendung der Synthesestrategie, wie in Tet. Let. 22, 1981, Seiten 1859 bis 1862, beschrieben, hergestellt werden. Die Oligonukleotide können dabei während des Herstellungsverfahrens entweder an der 5-oder der 3-Endstellung mit terminalen Aminogruppen derivatisiert werden. Eine weitere Möglichkeit der Anbindung solcher Fängermoleküle an die Innenwandoberflächen der Poren kann durchgeführt werden, indem das Substrat zunächst mit einer Chlorquelle, wie Cl₂, SOCl₂, COCl₂ oder (COCl)₂, gegebenfalls unter Verwendung eines Radikalinitiators wie Peroxide, Azoverbindungen oder Bu₃SnH, behandelt wird und anschließend mit einer entsprechenden nucleophilen Verbindung, wie insbesondere mit Oligonukleotiden bzw. DNA-Molekülen, die terminale primäre Aminogruppen oder Thiolgruppen aufweisen, umgesetzt werden (siehe WO 00/33976).

Die erfindungsgemäße Vorrichtung kann dann die Funktion eines 96-Probenträgers mit der Dichte eines Mikroarrays aufweisen. Weiterhin können auf Basis der erfindungsgemäßen Vorrichtung im Stand der Technik verfügbare Mikrochip-Technologien parallelisiert werden.

Die erfindungsgemäße Vorrichtung eignet sich insbesondere auch für die örtlich begrenzte, lichtgesteuerte Synthese von Molekülen an den Porenwänden. Ein weiterer Gegenstand der vorliegenden Erfindung betrifft somit ein Verfahren zum Steuern chemischer bzw. biochemischer Reaktionen bzw. Synthesen die Schritte:
- Bereitstellen einer erfindungsgemäßen Vorrichtung bzw. BioChips;
- Einleiten einer Synthesesubstanz in zumindest eine der Poren des Trägermaterials;
- Einkoppeln von Licht in die Pore zum optischen Anregen zumindest der Synthesesubstanz.

Für planare Substrate ist das Verfahren der lichtgesteuerten Synthese beispielsweise in EP 0 619 321 und EP 0 476 014 beschrieben. Auf den Offenbarungsgehalt dieser Druckschriften wird hinsichtlich des Aufbaus und des lichtgesteuerten Syntheseverfahren in vollem Umfang Bezug genommen, so daß diese Druckschriften insoweit zum Offenbarungsgehalt der vorliegenden Anmeldung gehören. Durch die effiziente Ausbreitung des Lichts in die Poren hinein können an den Porenwände photochemische Reaktionen getrieben bzw. gesteuert werden. Insbesondere können auf diese Weise komplexe sequentielle lichtgesteuerte chemische Reaktionen an den Porenbegrenzungsflächen ausgeführt werden.

Optisches Übersprechen zwischen den einzelnen Poren oder Bereichen/Kompartments wird durch die reflektierenden Wände aus im wesentlichen Silizium unterbunden. Damit wird ein Hauptproblem bei der lichtgesteuerten Synthese auf planaren Substraten gelöst.

Die vorliegende Erfindung wird nachfolgend unter Bezugnahme auf die Figuren beispielhaft beschrieben. Es zeigt:
- Fig. 1: (A) eine schematische Draufsicht und (B) eine Querschnittsansicht einer Ausführungsform einer erfindungsgemäßen Vorrichtung;
- Fig. 2: den Öffnungswinkel α, unter dem ein lumineszierendes Volumen (A) in einer konventionellen Pore, die vollständig von Siliziumwänden umgeben ist, und (B) in einer Pore einer erfindungsgemäßen Vorrichtung in den Raum über (und unter) dem Trägermaterial abstrahlen kann.

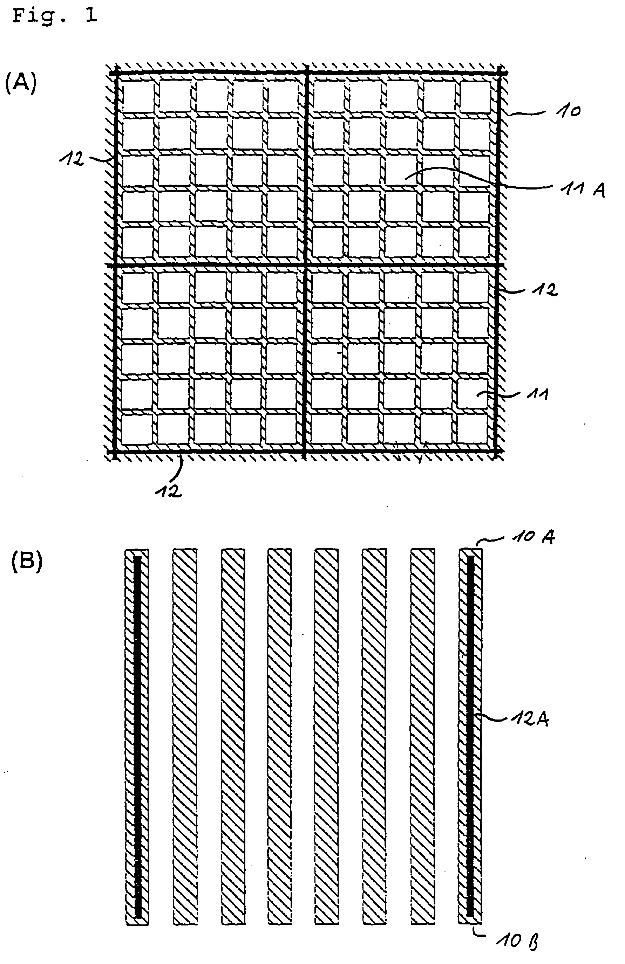

In Fig. 1 ist eine Ausführungsform einer erfindungsgemäßen Vorrichtung stark schematisiert zum einen in Draufsicht (Fig. 1(A)) und zum anderen im Querschnitt (Fig. 1(B)) gezeigt. Die erfindungsgemäße Vorrichtung weist dabei vollständig durchoxidierte Bereiche (11A) auf, die eine Vielzahl von im wesentlichen quadratischen Poren mit Porenwänden aus SiO₂ umfassen. Diese Bereiche (11A) sind von einem zu den Oberflächen (10A, 10B) hin offenen Rahmen (12) aus Wänden mit einem Siliziumkern (12A) eingefasst, wobei der Kern aus Silizium über den Querschnitt zur Außenseite der den Rahmen bildenden Wände hin in Siliziumdioxid übergeht.

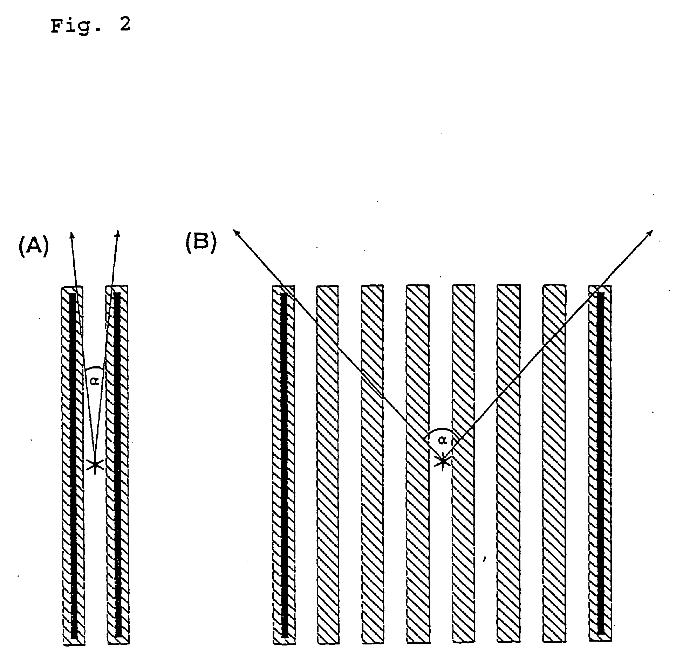

In Fig. 2 ist der Öffnungswinkel α gezeigt, unter dem ein lumineszierendes Volumen bzw. eine lumineszierende Fläche einer Pore einer erfindungsgemäßen Vorrichtung (Fig. 2(B)) in den Raum über (und unter) dem Substrat bzw. Trägermaterial abstrahlen kann, im Vergleich zu dem Fall in einer konventionellen Pore, die vollständig von Siliziumwänden umgeben ist (Fig. 2(A)). Der Öffnungswinkel, unter dem ein lumineszierendes Volumen bzw. eine lumineszierende Fläche in einer Pore in den Raum über (und unter) dem Substrat abstrahlen kann, ist in der erfindungsgemäßen Vorrichtung deutlich größer als in einer konventionellen Pore, die vollständig von Siliziumwänden umgeben ist. Durch das kleinere Aspektverhältnis (Öffnung oxidierter Bereich / Länge Poren) nimmt auch die Anzahl der nötigen Reflexionen ab, die ein Strahl unter einem gegebenen Winkel durchläuft, bis er die Öffnung der Poren erreicht. Im Vergleich zu einem porösen Siliziumsubstrat ohne transparente Bereiche bewirkt die erfindungsgemäße Vorrichtung eine erhebliche Verbesserung der absoluten Signalausbeute in Fluoreszenz- oder Chemilumineszenz-Analyseverfahren.

### Bezugszeichenliste

- 10: Trägermaterial auf Silizium-Basis
- 10A, 10B: Trägermaterialoberflächen
- 11: Pore
- 11A: Bereich, der ein oder mehrere Poren mit Porenwänden aus SiO₂ umfasst
- 12: Rahmen aus Wänden mit einem Siliziumkern
- 12A: Siliziumkern

## Patentansprüche

1. Vorrichtung, umfassend ein flächig ausgebildetes makroporöses Trägermaterial (10) auf Silizium-Basis, das über mindestens einen Oberflächenbereich verteilt eine Vielzahl von Poren (11) mit einem Durchmesser im Bereich von 500 nm bis 100 µm aufweist, welche sich von einer Oberfläche (10A) zur gegenüberliegenden Oberfläche (10B) des Trägermaterials durchgängig erstrecken, wobei die Vorrichtung mindestens einen Bereich (11A) aufweist, der mehrere Poren mit Porenwänden aus SiO₂ umfasst, und wobei dieser Bereich von einem zu den Längsachsen der Poren im wesentlichen parallel angeordneten, zu den Oberflächen (10A, 10B) hin offenen Rahmen (12) aus Wänden mit einem Siliziumkern (12A) umgeben ist, wobei der Kern aus Silizium über den Querschnitt zur Außenseite der den Rahmen bildenden Wände hin in Siliziumdioxid übergeht.

2. Vorrichtung nach Anspruch 1, wobei das Trägermaterial (10) eine Dicke zwischen 100 bis 5.000 µm aufweist.

3. Vorrichtung nach Anspruch 1 oder 2, wobei die Porendichte im Bereich von 10⁴ bis 10⁸/cm² liegt.

4. Vorrichtung nach einem der Ansprüche 1 bis 3, wobei die Poren (11) mit Porenwänden aus SiO₂ im wesentlichen quadratisch ausgebildet sind und der Rahmen (12) aus Wänden mit einem Siliziumkern (12A) in im wesentlichen quadratischer oder rechteckiger Form vorliegt.

5. Vorrichtung nach einem der vorangegangenen Ansprüche, wobei an zumindest einer der Poren (11) zumindest bereichsweise kovalent Fängermoleküle, ausgewählt aus der Gruppe, bestehend aus DNA, Proteinen und Liganden, gebunden sind.

6. Vorrichtung nach Anspruch 5, wobei die Fängermoleküle Oligonukleotidsonden sind, die über endständige Amino- oder Thiolgruppen an Linkermoleküle gebunden sind, die wiederum über kovalente und/oder ionische Gruppen an die Poren (11) gebunden sind.

7. Verfahren zur Herstellung einer Vorrichtung nach einem der Ansprüche 1 bis 6, umfassend die Schritte:
(a) Bereitstellen eines Trägermaterials (10) aus Silizium mit den Oberflächen (10A, 10B);
(b) Erzeugen von Sacklöchern, deren Tiefe geringer als die Dicke des Trägermaterials ist, durch elektrochemisches Ätzen in einer Oberfläche (10A) des Trägermaterials dergestalt, daß der Abstand der in einer ansonsten, im wesentlichen regelmäßigen Anordnung vorgesehenen Sacklöcher unter Ausbildung von Bereichsübergängen mit erhöhter Siliziumwanddicke bereichsweise geändert wird, wobei die Dicke der Siliziumwände zwischen den Bereichsübergängen um den Betrag des erhöhten Sacklöcherabstandes größer als die Dicke der Siliziumwände innerhalb der Bereiche gestaltet wird;
(c) mindestens bereichsweises Anordnen einer Maskenschicht auf der Oberfläche (10A) und der Oberfläche der in Schritt (b) erzeugten Sacklöcher;
(d) Abtragen des Trägermaterials mindestens bis zum Boden der Sacklöcher unter Erhalten von Poren (11), welche sich von einer Oberfläche (10A) zur gegenüberliegenden Oberfläche (10B) des Trägermaterials durchgängig erstrecken;
(e) Entfernen der Maskenschicht;
(f) Unterwerfen des in Schritt (e) erhaltenen Trägermaterials einer thermischen Oxidation, so daß in Abhängigkeit von der Siliziumwanddicke die Bereiche mit dünneren Silizium-Wänden vollständig oxidiert werden, während bei den Bereichsübergängen mit erhöhter Wanddicke die Siliziumwände nicht vollständig oxidiert werden, so daß ein Siliziumkern in den Wänden bestehen bleibt.

8. Verfahren nach Anspruch 7, wobei die Maskenschicht aus Si₃N₄ gebildet ist.

9. Verfahren nach Anspruch 7 oder 8, wobei das Abtragen in Schritt (d) durch Ätzen mit KOH oder durch einen CMP-Prozeß erfolgt.

10. Verfahren nach einem der Ansprüche 7 bis 9, worin die in Schritt (d) erzeugten Poren vor der Behandlung in Schritt (e) aufgeweitet bzw. vergrößert werden.

11. Verfahren nach Anspruch 10, wobei Si(100) als Trägermaterial eingesetzt wird und der Durchmesser der in Schritt (d) erzeugten Poren vor der Behandlung in Schritt (e) durch Ätzen in KOH unter Erhalten von im wesentlichen quadratischen Poren aufgeweitet wird.

12. Verwendung der Vorrichtung nach einem der Ansprüche 1 bis 6 als Basis für einen Probenträger in Verfahren zum Nachweis biochemischer Reaktionen und/oder Bindungen sowie hierfür insbesondere zur Untersuchung von enzymatischen Reaktionen, Nukleinsäure-Hybridisierungen, Protein-Protein-Wechselwirkungen und Protein-Liganden-Wechselwirkungen.

13. Verfahren zum Steuern chemischer bzw. biochemischer Reaktionen bzw. Synthesen, umfassend die Schritte:
- Bereitstellen einer Vorrichtung nach einem der vorhergehenden Ansprüche 1 bis 6;
- Einleiten einer Synthesesubstanz in zumindest eine der Poren des Trägermaterials;
- Einkoppeln von Licht in die Pore zum optischen Anregen zumindest der Synthesesubstanz.

## Claims

1. Device comprising a flatly designed macroporous support material (10) based on silicon, which has a multiplicity of pores (11) with a diameter in the range of from 500 nm to 100 µm distributed over at least one surface region and extending from one surface (10A) through to the opposite surface (10B) of the support material, wherein the device has at least one region (11A) which comprises more pores with SiO₂ pore walls, and wherein this region is surrounded by a frame (12) of walls with a silicon core (12A) which is arranged essentially parallel to the longitudinal axes of the pores and is open towards the surfaces (10A, 10B), wherein the silicon core merges into silicon dioxide over the cross section towards the outer side of the walls forming the frame.

2. Device according to Claim 1, wherein the support material (10) has a thickness between 100 to 5000 µm.

3. Device according to Claim 1 or 2, wherein the pore density is in the range of from 10⁴ to 10⁸/cm².

4. Device according to one of Claims 1 to 3, wherein the pores (11) with SiO₂ pore walls are designed essentially squarely and the frame (12) of walls with a silicon core (12A) is in an essentially square or rectangular shape.

5. Device according to one of the preceding claims, wherein capture molecules selected from the group consisting of DNA, proteins and ligands are covalently bound at least locally to at least one of the pores (11).

6. Device according to Claim 5, wherein the capture molecules are oligonucleotide probes which are bound via terminal amino or thiol groups to linker molecules, which are in turn bound to the pores (11) via covalent and/or ionic groups.

7. Method for the production of a device according to one of Claims 1 turn 6, comprising the following steps:
(a) preparing a support material (10) made of silicon with the surfaces (10A, 10B);
(b) producing blind holes whose depth is less than the thickness of the support material by electrochemical etching into one surface (10A) of the support material, in such a way that the spacing of the blind holes provided in an otherwise essentially regular arrangement is locally modified to form inter-region transitions with an increased silicon wall thickness, wherein the thickness of the silicon walls between the inter-region transitions is configured to be greater than the thickness of the silicon walls inside the region by the amount of the increased blind-hole spacing;
(c) at least locally arranging a mask layer on the surface (10A) and the surface of the blind holes produced in step (b);
(d) eroding the support material at least as far as the bottom of the blind holes in order to obtain pores (11) which extend from one surface (10A) through to the opposite surface (10B) of the support material;
(e) removing the mask layer;
(f) subjecting the support material obtained in step (e) to a thermal oxidation so that, as a function of the silicon wall thickness, the regions with thinner silicon walls are fully oxidized whereas the silicon walls are not fully oxidized in the inter-region transitions with an increased wall thickness, so that a silicon core is left remaining in the walls.

8. Method according to Claim 7, wherein the mask layer is formed by Si₃N₄.

9. Method according to Claim 7 or 8, wherein the erosion in step (d) is carried out by etching with KOH or by a CMP process.

10. Method according to one of Claims 7 to 9, in which the pores produced in step (d) are widened or enlarged before the treatment in step (e).

11. Method according to Claim 10, wherein Si(100) is used as the support material and the diameter of the pores produced in step (d) is widened by etching in KOH before the treatment in step (e) in order to obtain essentially square pores.

12. Use of the device according to one of Claims 1 to 6 as a basis for a sample support in methods for detecting biochemical reactions and/or bindings and, in this context, in particular for the study of enzymatic reactions, nucleic acid hybridizations, protein-protein interactions and protein-ligand interactions.

13. Method for controlling chemical or biochemical reactions or syntheses, comprising the following steps:
- preparing a device according to one of the preceding Claims 1 to 6;
- introducing a synthesis substance into at least one of the pores of the support material;
- shining light into the pores in order to optically excite at least the synthesis substance.

## Revendications

1. Dispositif comportant un matériau support macroporeux (10), bidime6nsionnel, à base de silicium, qui comprend, répartis sur au moins une zone de sa surface, un grand nombre de pores (11) ayant un diamètre compris dans la plage de 500 nm à 100 µm, qui s'étendent en continu d'une surface (10A) à la surface opposée (10B) du matériau support, le dispositif comportant au moins une zone (11A) qui possède plusieurs pores ayant des parois en SiO₂, cette zone étant entourée d'un cadre (12), disposé pour l'essentiel parallèlement aux axes longitudinaux des pores, et ouvert vers les surfaces (10A, 10B), ce cadre étant constitué de parois ayant un coeur de silicium (12A), le coeur de silicium s'étendant, en section transversale, jusqu'à la face extérieure des parois formant le cadre, pour pénétrer dans le dioxyde de silicium.

2. Dispositif selon la revendication 1, dans lequel le matériau support (10) a une épaisseur comprise entre 100 et 5000 µm.

3. Dispositif selon la revendication 1 ou 2, dans lequel la densité des pores est comprise dans la plage de 10⁴ à 10⁸/cm².

4. Dispositif selon l'une des revendications 1 à 3, dans lequel les pores (11), ayant des parois en SiO₂, ont pour l'essentiel une forme carrée, et le cadre (12), constitué de parois ayant un coeur de silicium (12A), est présent sous une forme essentiellement carrée ou rectangulaire.

5. Dispositif selon l'une des revendications précédentes, dans lequel des molécules captrices, choisies dans l'ensemble consistant en l'ADN, les protéines et les ligands, sont, au moins par zones, liées par liaison covalente à au moins l'un des pores (11).

6. Dispositif selon la revendication 5, dans lequel les molécules captrices sont des sondes oligonucléotidiques, qui sont liées à des molécules lieuses par l'intermédiaire de groupes amino ou thiol terminaux, molécules qui, pour leur part, sont liées aux pores (11) par l'intermédiaire de groupes covalents et/ou ioniques.

7. Procédé de fabrication d'un dispositif selon l'une des revendications 1 à 6, comprenant les étapes consistant :
(a) à mettre à disposition un matériau support (10) en silicium, avec les surfaces (10A, 10B) ;
(b) à produire des trous borgnes, dont la profondeur est inférieure à l'épaisseur du matériau support, par gravure électrochimique dans une surface (10A) du matériau support, de telle sorte que la distance entre les trous borgnes, qui sont prévus selon un arrangement par ailleurs pour l'essentiel régulier, subit une variation par zones, avec formation de transitions de zones présentant une épaisseur accrue de paroi de silicium, l'épaisseur des parois de silicium entre les transitions de zones étant, de la distance ainsi augmentée entre les trous borgnes, supérieure à l'épaisseur des parois de silicium à l'intérieur des zones ;
(c) au moins par zones, à disposer une couche formant masque sur la surface (10A) et sur la surface des trous borgnes produits dans l'étape (b) ;
(d) à enlever le matériau support, au moins jusqu'au fond des trous borgnes, en obtenant des pores (11) qui s'étendent en continu d'une surface (10A) jusqu'à la surface opposée (10B) du matériau support ;
(e) à enlever la couche formant masque ;
(f) à soumettre le matériau support obtenu dans l'étape (e) à une oxydation thermique de telle sorte que, en fonction de l'épaisseur de la paroi de silicium, les zones ayant des parois de silicium plus minces subissent une oxydation complète, tandis que les parois de silicium ne vont pas subir une oxydation complète dans les transitions de zone ayant une épaisseur de paroi accrue, de sorte qu'il reste dans les parois un coeur de silicium.

8. Procédé selon la revendication 7, dans lequel la couche formant masque est formée de Si₃N₄.

9. Procédé selon la revendication 7 ou 8, dans lequel l'enlèvement de l'étape (d) est réalisé par gravure avec KOH, ou par un procédé CMP.

10. Procédé selon l'une des revendications 7 à 9, dans lequel les pores produits dans l'étape (d) subissent, avant le traitement de l'étape (e), un élargissement ou un agrandissement.

11. Procédé selon la revendication 10, dans lequel on utilise en tant que matériau support du Si(100), et le diamètre des pores produits dans l'étape (d) subit, avant le traitement de l'étape (e), un élargissement par gravure dans du KOH, avec obtention de pores essentiellement carrés.

12. Utilisation du dispositif selon l'une des revendications 1 à 6 en tant que base pour un porte-échantillon dans un procédé destiné à détecter des réactions biochimiques et/ou des liaisons, et aussi, dans ce cas, destiné notamment à étudier des réactions enzymatiques, des hybridations d'acide nucléique, des interactions protéine-protéine et des interactions protéine-ligand.

13. Procédé pour piloter des réactions ou des synthèses chimiques ou biochimiques, comprenant les étapes consistant :
- à mettre à disposition un dispositif selon l'une des revendications précédentes 1 à 6 ;
- à introduire une substance de synthèse dans au moins l'un des pores du matériau support ;
- à injecter de la lumière dans les pores, pour excitation optique d'au moins la substance de synthèse.
